# EUROPEAN PATENT APPLICATION

(11) **EP 2 596 752 A1**
(43) Date of publication of application: **29.05.2013**
(21) Application number: 12194114.0
(22) Date of filing: 23.11.2012
(51) Int. Cl.: A61B 6/04, G01D 5/347, G06K 19/06, H03M 1/24

(54) **Medical imaging apparatus**

(30) Priority: 24.11.2011 KR 20110123509
(71) Applicant: Samsung Electronics Co., Ltd., Suwon-si, Gyeonggi-do 442-742 (KR)
(72) Inventor: Lee, Ho Jun, Suwon-si Gyeonggi-do (KR)
(74) Representative: Haan, Raimond

(57) **Abstract**

A medical imaging apparatus provided with a position recognition member, capable of achieving a precise position recognition without an additional calibration process and preventing the position recognition errors on account of a long term use of components includes an X-ray generating portion configured to generate X-rays and transmit the X-rays, an X-ray detecting portion configured to detect the X-rays transmitted from the X-ray generating portion, a moving portion provided on at least one of the X-ray generating portion and the X-ray detecting portion to move the at least one of the X-ray generating portion and the X-ray detecting portion, and a position recognition member comprising a bar code portion configured to recognize a position of the at least one of the X-ray generating portion and the X-ray detecting portion, which move along the moving portion, and a bar code reader portion to recognize the bar code portion.

## Description

### BACKGROUND

### 1. Field

One or more embodiments relate to a medical imaging apparatus, and more particularly, to a medical imaging apparatus provided with a position recognition member.

### 2. Description of the Related Art

In general, a medical imaging apparatus is designed to obtain an internal image of a human body by use of electromagnetic radiation in the form of X-ray. The medical imaging apparatus obtains an internal image of a human body by projecting X-ray toward a desired region of the human body, such as the head or the breast, and by detecting the X-ray passing through the desired region.

The medical imaging apparatus is classified into a plain X-ray imaging apparatus that transmits X-rays in one direction, and a Computed Tomography (CT) imaging apparatus that reconstructs a computed image by transmitting X-rays in a plurality of directions. The CT imaging apparatus may be referred to as a computed tomography apparatus or a computerization tomography apparatus.

The medical imaging apparatus includes an X-ray tube to project X-ray to a region of the human body, a high voltage generator to generate a high voltage that is required for generating the X-ray, and a moving apparatus to move a projecting position and a projecting direction of the X-ray. In addition, the medical imaging apparatus is provided with a manipulating apparatus configured for an operator to manipulate the control of the apparatuses of the medical imaging apparatus.

The medical imaging apparatus receives X-rays, obtains a medical image and transmits the obtained medical image to a server or a communication device. The medical imaging apparatus receives projected X-rays, changes the received X-rays to digital signals and transmits digital data to a personal computer. The medical imaging apparatus includes an X-ray detecting portion to detect X-rays. The X-ray detecting portion may be installed on a stand that is configured to obtain an X-ray image while having a patient in a standing position, or a patient table that is configured to obtain an X-ray image while having a patient in a laid position. The X-ray receiving portion may be provided in a fixed state, or in recent years, in a detachable manner provided for the use of both of the stand or the patient table.

In recent years, the medical apparatus is provided in the shape of U-arm having an X-ray detecting portion and an X-ray generating portion incorporated into a unitary apparatus. The medical imaging apparatus as such offers easy manipulation without having spatial limitation.

The medical imaging apparatus requires an apparatus that is configured to recognize the position of the X-ray detecting portion or the X-ray generating portion to control the movement of the X-ray detecting portion or the X-ray generating portion. In order to the recognize the position of the X-ray detecting portion or the X-ray generating portion, a relative coordinate system is used in a manner to detect a moving distance from a reference point such that the current position is detected. In the case where the relative coordinate system is used, POT or an absolute encoder is used to detect the moving distance.

However, when the POT or the absolute encoder is used for a long period of time, the accuracy of encoding coordinates may be lowered due to the deterioration of the components. In addition, for the relative coordinate system, a calibration process is needed to set a reference point. If the calibration is not accurate, the whole coordinate system may have errors.

### SUMMARY

The foregoing described problems may be overcome and/or other aspects may be achieved by one or more embodiments of a medical imaging apparatus having a position recognition member.

Additional aspects and/or advantages of one or more embodiments will be set forth in part in the description which follows and, in part, will be apparent from the description, or may be learned by practice of one or more embodiments of disclosure. One or more embodiments are inclusive of such additional aspects.

In accordance with one or more embodiments, a medical imaging apparatus may include an X-ray generating portion, an X-ray detecting portion, a moving portion, and a position recognition member. The X-ray generating portion may generate X-rays and transmit the X-rays. The X-ray detecting portion may detect the X-rays transmitted from the X-ray generating portion. The moving portion may be provided on at least one of the X-ray generating portion and the X-ray detecting portion to move the at least one of the X-ray generating portion and the X-ray detecting portion. The position recognition member may include a bar code portion configured to recognize a position of the at least one of the X-ray generating portion and the X-ray detecting portion, which may move along the moving portion, and a bar code reader portion to recognize the bar code portion.

The bar code portion and the bar code reader portion may be provided to face each other.

The bar code reader portion may be formed on a protrusion that protrudes from at least one portion of the medical imaging apparatus, the at least one portion facing the bar code portion.

The bar code reader portion may be formed at an inside of at least one portion of the medical imaging apparatus, the at least one portion facing the bar code portion.

The medical imaging apparatus, which may accommodate the bar code reader portion, may include transparent material that allows light, which is transmitted from the bar code reader portion, to pass therethrough.

The X-ray generating portion may include a direction adjusting portion configured to adjust a direction in which the X-ray is transmitted toward an object. The direction adjusting portion may include a guide rail and a moving carriage that is disposed at a lower side of the guide rail to move along the guide rail.

The moving portion may be provided on the guide rail.

The guide rail may include a first guide rail and a second guide rail, the bar code portion may be positioned on at least one of the first guide rail and the second guide rail, and the bar code reader portion to recognize the bar code portion may be provided at an upper side of the moving carriage, which may face the at least one of the first guide rail and the second guide rail.

The guide rail may include a first guide rail and a second guide rail that are disposed while being in perpendicular to each other.

The medical imaging apparatus may further include a driving apparatus configured to support one of the first guide rail and the second guide rail against a remaining one of the first guide rail and the second guide rail while driving the remaining one of the first guide rail and the second guide rail with respect to the one of the first guide rail and the second guide rail. Each of the first guide rail and the second guide rail may be provided in one pair, a first bar code portion may be provided at one side of the first guide rail and a first bar code reader portion to recognize the first bar code portion may be provided at one side of the moving carriage.

A second bar code portion may be provided at one side of the second guide rail and a second bar code reader portion to recognize the second bar code portion may be provided at another side of the moving carriage.

The medical imaging apparatus may further include a table configured to have the object disposed thereon, the table including a table top on which the object is disposed and a support to support the table top. The X-ray detecting portion may be provided at a lower side of the table top.

The moving portion may be disposed at the lower side of the table top to move the X-ray detecting portion.

The bar code portion may be provided at an edge of the table and the bar code reader portion may be provided at one side of the X-ray detecting portion.

The bar code portion may have a width of about 0.1 to 1mm.

In accordance with one or more embodiments, a medical imaging apparatus may include an X-ray generating portion, a stand, a moving portion and a position recognition member. The X-ray generating portion may be configured to generate X-rays and transmit the X-rays. The stand may include an X-ray detecting portion to detect the X-ray transmitted from the X-ray generating portion and a body portion while having the X-ray detecting portion movably coupled to the body portion. The moving portion may be provided on at least one of the X-ray generating portion and the X-ray detecting portion to move the at least one of the X-ray generating portion and the X-ray detecting portion. The position recognition member may include a bar code portion configured to recognize a position of the at least one of the X-ray generating portion and the X-ray detecting portion, which move along the moving portion, and a bar code reader portion to recognize the bar code portion.

The moving portion may be mounted on the body portion to move the X-ray detecting portion upward and downward, the bar code portion may be provided on one surface of the body portion facing the X-ray detecting portion, and the bar code reader portion may be provided at a rear surface of the X-ray detecting portion.

In accordance with one or more embodiments, a medical imaging apparatus may include an X-ray generating portion, an X-ray detecting portion, an arm portion, a moving portion, and a position recognition member. The X-ray generating portion may be configured to generate X-rays and transmit the X-rays. The X-ray detecting portion may be configured to detect the X-rays transmitted from the X-ray generating portion. The arm portion may enable the X-ray detecting portion and the X-ray generating portion to be installed in one apparatus. The moving portion may be provided on at least one of the X-ray generating portion and the X-ray detecting portion to move the at least one of the X-ray generating portion and the X-ray detecting portion. The position recognition member may include a bar code portion configured to recognize a position of the at least one of the X-ray generating portion and the X-ray detecting portion, which move along the moving portion, and a bar code reader portion to recognize the bar code portion.

The medical imaging apparatus may further include a body portion configured to support the arm portion, which connects the X-ray detecting portion to the X-ray generating portion.

The arm portion may include a first arm portion coupled to the body portion, and a second arm portion extending from the first arm portion to the X-ray detecting portion.

The bar code portion may be provided at one side of the body portion facing the second arm portion, and the bar code reader portion may be provided at one side of the second arm portion facing the body portion.

The bar code portion may be provided at one surface of the first arm portion to which the X-ray generating portion is coupled, and the bar code reader portion may be provided at a rear side of the X-ray generating portion.

In accordance with one or more embodiments, a medical imaging apparatus may include a gantry, a table and a moving portion. The gantry may be provided at a center thereof with a round tunnel to accommodate an object while being configured to rotate around the object. The gantry may include an X-ray generating portion, an X-ray detecting portion, and a position recognition member. The X-ray generating portion may generate X-rays at an inner side of the gantry and transmit the X-rays. The X-ray detecting portion may detect the X-rays received from the -ray generating portion. The position recognition member may include a first bar code portion to recognize coordinates of the X-ray generating portion and a first bar code reader portion to recognize the first bar code portion. The table may have the object disposed thereon, and include a transport portion to transport the object to an inside of the gantry and a support potion to support the table. The moving portion may be provided on at least one of the table and the inner side of the gantry.

The first bar code portion may surround the inner side of the gantry in a circle.

The first bar code reader portion may be provided at a rear side of the X-ray detecting portion that faces an inner wall of the gantry.

The first bar code reader portion may be provided at a rear side of the X-ray generating portion that faces an inner wall of the gantry.

The table may include a second bar code portion provided at one side of the transport portion to recognize a position of the object, and a second bar code reader portion provided at the support portion to recognize the second bar code portion.

As described above, the accuracy in the position recognition may be improved without an additional calibration. In addition, the position recognition may be achieved based on optical characteristics, thereby possibly preventing position recognition errors due to a long term use of components.

### BRIEF DESCRIPTION OF THE DRAWINGS

These and/or other aspects of the disclosure will become apparent and more readily appreciated from the following description of embodiments, taken in conjunction with the accompanying drawings of which:
FIG. 1 is a perspective view illustrating a position recognition member in accordance with one or more embodiments.
FIG. 2 is an enlarged view illustrating a barcode portion in accordance with one or more embodiments.
FIG. 3 is a view illustrating a medical imaging apparatus in accordance with one or more embodiments.
FIG. 4 is an enlarged view illustrating an X-ray generating portion in accordance with one or more embodiments.
FIG. 5 is a view illustrating a table in accordance with one or more embodiments.
FIG. 6 is a view illustrating a stand in accordance with one or more embodiments.
FIG. 7 is a view illustrating a medical imaging apparatus in accordance with one or more embodiments.
FIG. 8 is a view illustrating a medical imaging apparatus in accordance with one or more embodiments.
FIG. 9 is a view illustrating the interior of a medical imaging apparatus in accordance with one or more embodiments.

### DETAILED DESCRIPTION

Reference will now be made in detail to one or more embodiments, illustrated in the accompanying drawings, wherein like reference numerals refer to like elements throughout. In this regard, embodiments of the present invention may be embodied in many different forms and should not be construed as being limited to embodiments set forth herein, as various changes, modifications, and equivalents of the systems, apparatuses and/or methods described herein will be understood to be included in the invention by those of ordinary skill in the art after embodiments discussed herein are understood. Accordingly, embodiments are merely described below, by referring to the figures, to explain aspects of the present invention.

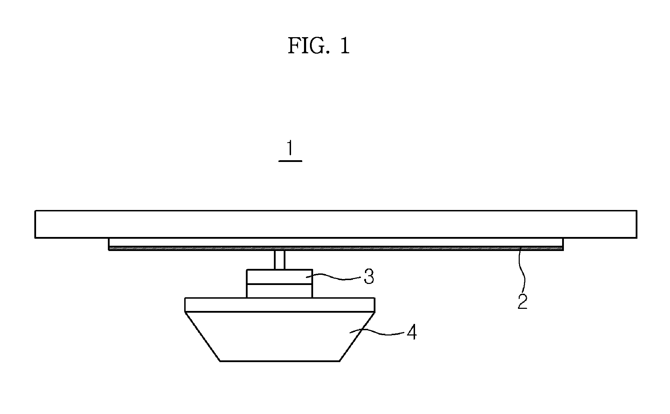

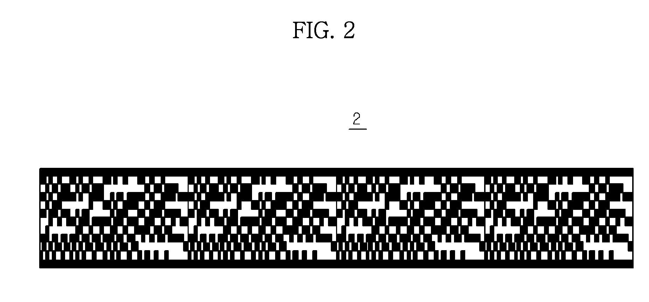

FIG. 1 is a perspective view illustrating a position recognition member in accordance with one or more embodiments. FIG. 2 is an enlarged view illustrating a bar code portion in accordance with one or more embodiments.

A medical imaging apparatus may include a position recognition member 1 shown in FIGS. 1 and 2. The position recognition member 1 may be provided on at least one of an electromagnetic radiation generating portion and an electromagnetic radiation detecting portion. In one or more example embodiments, the electromagnetic radiation may be X-rays. The position recognition member 1 may include a bar code portion 2 to recognize the position of at least one of the X-ray generating portion and the X-ray detecting portion, and a bar code reader portion 3 to recognize the bar code portion 2.

The bar code portion may be positioned on at least one of the X-ray generating portion and the X-ray detecting portion. The medical imaging apparatus may include a moving portion provided on at least one of the X-ray generating portion and the X-ray detecting portion to move at least one of the X-ray generating portion and the X-ray detecting portion. The moving portion may include a configuration to move the X-ray generating portion or the X-ray detecting portion. For example, the moving portion may be implemented using a rail. The bar code 2 may be provided to recognize the position of at least one of the X-ray generating portion and the X-ray detecting portion that move along the moving portion.

The bar code reader portion 3 may be provided therein with an optical sensor to recognize the position of at least one of the X-ray generating portion and the X-ray detecting portion by recognizing the bar code portion 2 through the optical sensor. The bar code portion 2 may be disposed to be facing the bar code reader portion 3.

As shown in FIG. 2, the bar code portion 2 may be provided in the form of a combination of different widths of bars including numerical information enabling to recognize the position of at least one of the X-ray generating portion and the X-ray detecting portion. The bar code reader 3 including the optical sensor corresponding to the bar code portion 2 may recognize the position of at least one of the X-ray generating portion and the X-ray detecting portion by transmitting light and reading an electronic signal that is generated according to the strength and weakness of reflected light.

The bar code portion 2 may be provided to allow the position of the moving portion, which may be provided on the X-ray generating portion or the X-ray detecting portion, to be recognized. Accordingly, the bar code portion may be provided on the moving portion, or on a portion facing the moving portion. Although the bar code portion 2 may have a width of about 0.1 to 1mm that enables the bar code reader portion 3 to recognize the bar code portion 2, the width of the bar code portion 2 is not limited thereto. In addition, the bar code portion 2 may be formed through a printing scheme, but not limited thereto.

In order to the bar code reader portion 3 to recognize the bar code portion 2, the bar code reader portion 3 may be positioned on a protrusion 4 that may protrude on at least one portion of the medical imaging apparatus. However, the position of the bar code reader portion 3 is not limited thereto, and the bar code reader portion 3 may be positioned at an inner side of the medical imaging apparatus. In the case where the bar code reader portion 3 is positioned at an inner side of the medical imaging apparatus, an outer side of the medical imaging apparatus may be made of transparent material that allows light, which may be transmitted from the bar code reader portion 3, to pass therethrough

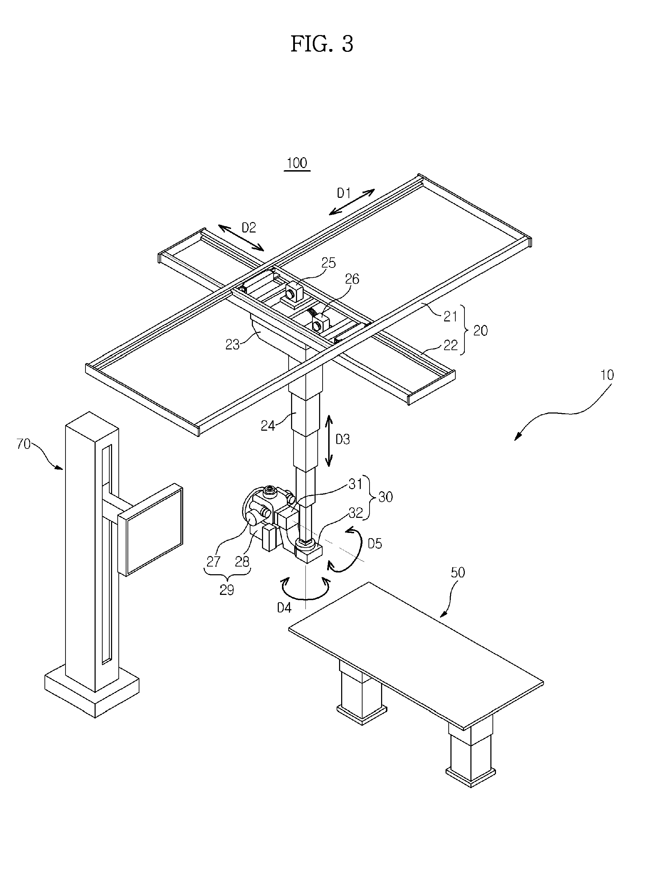

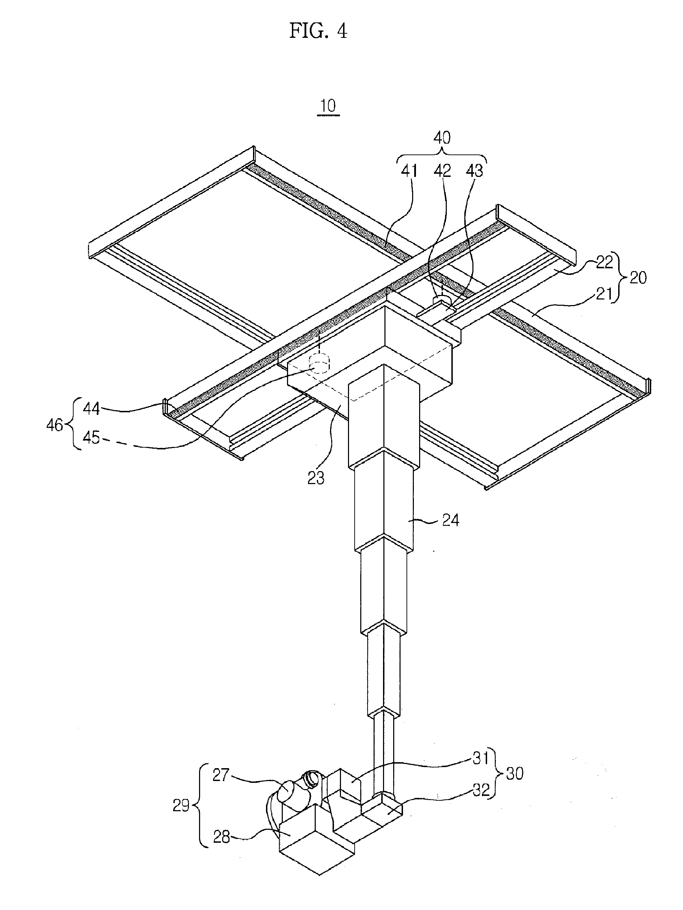

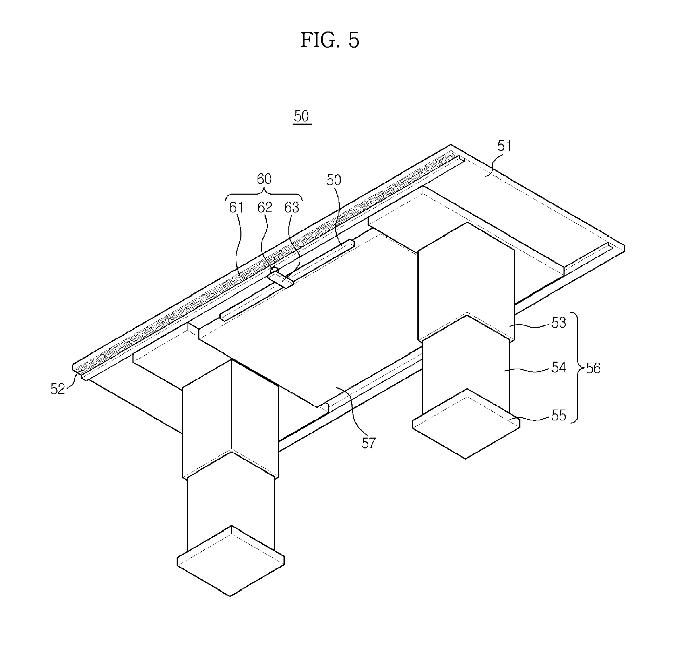

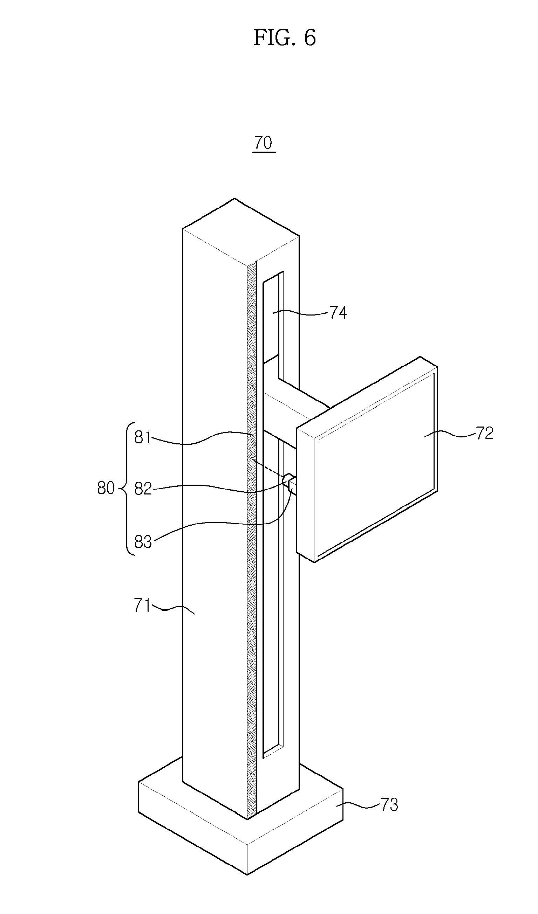

FIG. 3 is a view illustrating a medical imaging apparatus in accordance with one or more embodiments. FIG. 4 is an enlarged view illustrating an X-ray generating portion in accordance with one or more embodiments. FIG. 5 is a view illustrating a table in accordance with one or more embodiments. FIG. 6 is a view illustrating a stand in accordance with one or more embodiments. The medical imaging apparatus may be applied to various types of medical imaging apparatus, but the following description will be made in relation to a medical imaging apparatus having a guide rail installed at a ceiling of an examination room.

Referring to FIGS. 3 and 6, a medical imaging apparatus 100 may include an X-ray generating portion 10, a table 50 on which an object is disposed, and a stand 70. The X-ray generating portion 10 may include an imaging portion 29 and a direction adjusting portion.

The X-ray generating portion 10 may include a direction adjusting portion to transmit X-rays toward the object. The direction adjusting portion may include a guide rail 20 and a moving carriage 23 that is disposed at a lower side of the guide rail 20 to move along the guide rail 20. In addition, the X-ray generating portion 10 may include a post frame 24 and driving apparatuses 25 and 26.

The guide rail 20 may include a first guide rail 21 and a second guide rail 22. The first guide rail 21 and the second guide rail 22 may extend while being perpendicular to each other.

The first guide rail 21 may be installed, for example, on a ceiling of an examination room in which the medical imaging apparatus 100 is disposed. The second guide rail 22 may be disposed at a lower side of the first guide rail 21 while being installed in a slidable manner on the first guide rail 21. The first guide rail 21 may be provided with a roller (not shown) installed thereto that may be movable along the first guide rail 21. The second guide rail 22 may move along the first guide rail 21 while being connected to the roller.

The direction to which the first guide rail 21 extends is designated as a first direction D1, and the direction to which the second guide rail 22 extends is designated as a second direction D2. Accordingly, the first direction D1 and the second direction D2 may be parallel to the examination room while being in perpendicular to each other.

Although the moving carriage 23 according to one or more embodiments may be disposed at a lower side of the second guide rail 22 so as to move along the second guide rail 22, the disposition of the moving carriage 23 is not limited thereto. The moving carriage 23 may be provided at a lower side of one of the first guide rail 21 and the second guide rail 22, the one disposed at a lower side of the remaining. The moving carriage 23 may be provided with a roller (not shown) that moves along the second guide rail 22. Accordingly, the moving carriage 23 may be movable together with the second guide rail 22 in the first direction D1, and may be movable along the second guide rail 22 in the second direction D2.

The post frame 24 may be disposed at a lower side of the moving carriage 23 while being fixed to the moving carriage 23. The post frame 24 may be provided with a plurality of posts. The plurality of posts may be connected in a foldable manner to each other so that the post frame 24 may be increased or decreased lengthways in the upward/downward direction of the examination room while being fixed to the moving carriage 23. The direction in which the post frame 24 may be increased or decreased lengthways is designated as a third direction D3. Accordingly, the third direction D3 may be perpendicular to the first direction D1 and the second direction D2.

The imaging portion 29 may be configured to project X-rays toward an object. The imaging portion 29 may represent an X-ray tube that is typically provided in a general medical imaging apparatus. The X-ray tube may be provided with an X-ray generating source 27 to generated X-rays, and a collimator 28 to guide the generated X-ray toward the object.

A rotating joint 30 may be disposed between the imaging portion 29 and the post frame 24. The rotating joint 30 may be configured to couple the imaging portion 29 to the post frame 24 and to support a load acting on the imaging portion 29.

The rotating joint 30 may be provided with a first rotating joint 31 connected to a lower end of the post frame 24, and with a second rotating joint 32 connected to the imaging portion 29. The first rotating joint 31 may be provided so as to rotate around a central shaft of the post frame 24 that may extend in the upward/downward direction of the examination room. Accordingly, the first rotating joint 31 may be rotatable on a plane which is provided in perpendicular to the third direction D3. In this case, the direction in which the first rotating joint 31 may be rotated is designated as a fourth direction D4. The fourth direction D4 may correspond to a rotation direction of a shaft which may be provided in parallel to the third direction D3.

The second rotating joint 32 may be provided to rotate on a plane which is perpendicular to the ceiling of the examination room. Accordingly, the second rotating joint 32 may rotate in a rotation direction of a shaft which may be provided in parallel to the first direction D1 or the second direction D2. In this case, the direction in which the second rotating joint 32 may be rotated is designated as a fifth direction D5. The fifth direction D5 may correspond to a rotation direction of a shaft which may extend in the first direction D1 or the second direction D2.

The imaging portion 29 may be rotated in the fourth direction D4 and the fifth direction D5 while being connected to the rotating joint 30. In addition, the imaging portion 29 may linearly move in the first direction D1, the second direction D2 and the third direction D3 while being connected to the post frame 24 through the rotating joint 30.

In order to move the imaging portion 29 in the first to fifth directions D1 to D5, the driving apparatuses 25 and 26 may be provided. The driving apparatus may be a motor that is operated on electricity.

The driving apparatuses 25 and 26 may be disposed at various locations depending on convenience of design. For example, the first driving apparatus 26 to move the second guide rail 22 in first direction D1 may be disposed around the first guide rail 21, and the second driving apparatus 25 to move the moving carriage 23 in the second direction D2 may be disposed around the second guide rail 22. In addition, additional driving apparatuses may be provided to change the length of the post frame 24 and rotate the X-ray generating portion.

Each of the driving apparatuses 25 and 26 may be connected to a power transmission member (not shown) to allow the imaging portion 29 to rotate or linearly move in the first to fifth directions D1 to D5. The power transmission member may include a belt, a pulley, a chain, a sprocket, and/or a shaft.

Referring to FIG. 4, the X-ray generating portion 10 may be provided with position recognition members 40 and 46. A moving portion may be provided on the guide rail 20. The X-ray generating portion 10 may move along with the moving portion.

Bar code portions 41 and 44 may be positioned on at least one of the first guide rail 21 and the second guide rail 22. The first guide rail 21 and the second guide rail 22 may be provided in a pair to enable a smooth movement and to support the X-ray generating portion 10. The bar code portions 41 and 44 may be attached to at least one of the first guide rail 21 and the second guide rail 22, for example.

A bar code portion may be attached to a lower side of the first guide rail 21, and may be referred to as a first bar code portion 41. In order to recognize the first barcode portion 41, a bar code reader portion may be disposed at an upper side of the moving carriage 23, and may be referred to as a first bar code reader portion 42. The first bar code reader portion 42 may be provided on a first protrusion 43 that protrudes from the moving carriage 23 to an outer side of the moving carriage 23 to recognize the first bar code portion 41. The first bar code portion 41 and the first bar code reader portion 42 may be referred to as a first position recognition member 40 in cooperation with each other to recognize the position of the X-ray generating portion 10. The first position recognition member 40 may recognize coordinates when the X-ray generating portion 10 moves in the first direction D1.

The bar code portion may be attached to a lower side of the second guide rail 22, and may be referred to as a second bar code portion 44. In order to recognize the second bar code portion 44, a bar code reader portion may be disposed at an upper side of the moving carriage 23, and may be referred to as a second bar code reader portion 45. The second bar code reader portion 45 may be provided on a second protrusion (not shown) that protrudes toward the ceiling from the moving carriage 23 to recognize the second bar code portion 44. The second bar code portion 44 and the second bar code reader portion 45 may be referred to as a second position recognition member 46 in cooperation with each other to recognize the position of the X-ray generating portion 10. The second position recognition member 46 may recognize coordinates when the X-ray generating portion 10 moves in the second direction D2.

Referring to FIG. 5, a position recognition member 60 may be provided on the table 50. The table 50 may include a table top 51 on which an object may be disposed, and a frame portion 52 to support the table top 51. A support portion 56 to support the table top 51 against the floor may be provided at a lower side of the table top 51.

The support portion 56 may be provided with a plurality of supports 53 and 54 installed thereon in a foldable manner to adjust the height of the table 50. In addition, the support portion 56 may include a bottom surface 55 to support the plurality of supports 54 and 54 while making contact with the floor. In addition, the support 56 may include a configuration to drive the table 50. However, the shape of the support 56 is not limited thereto, and the support may be provided in other shapes capable of supporting the table top 51 against the floor.

Since the table top 51 has an X-ray detecting portion 57 disposed at a lower side thereof, the table top 51 may be formed using material having a low absorption rate of X-ray such that transmission of X-ray may be maximized while minimizing the absorption of X-ray. In general, the table top 57 is formed using wood, acryl, and/or carbon, for example.

A patient handle portion is a portion by which an object grips during the examination. The patient handle portion may be detachably installed on the frame portion 52.

The position recognition member 60 may be provided at a lower side of the table top 51. The X-ray detecting portion 57 may be movable in upside/downside and left/right directions at a lower side of the table top 51. Accordingly, the moving portion may be provided at a lower side of the table top 51 to move the X-ray detecting portion 57. A bar code 61 may be disposed at a lower side of the frame portion 52 of the table 50. A bar code reader portion 62 may be disposed at the moving portion facing the bar code portion 61. The bar code reader portion 62 may be provided at a protrusion that protrudes from the X-ray detecting portion 57, but the disposition of the bar code reader portion 62 is not limited thereto. The bar code reader portion 62 may be provided at an upper side of the X-ray detecting portion 57 according to the disposition of the barcode portion 61 to recognize the barcode portion 61. In the case where the bar code reader portion 62 is provided at the upper side of the X-ray detecting portion 57, the upper side of the X-ray detecting portion 57 may be formed using transparent material allowing light to pass therethrough.

By use of the position recognition member 60, the movement of the X-ray detecting portion 57 may be detected as the bar code reader portion 62 scans the bar code portion 61, and thus the precise coordinates of the X-ray detecting portion 57 may be obtained.

Referring to FIG. 6, a position recognition member 80 may be provided on a stand 70. The stand 70 may include an X-ray detecting portion 72 and a body portion 71. In order for the X-ray detecting portion 72 to be coupled to the body portion 71 so as to enable an upward and downward motion, a moving portion 74 may be provided on the body portion 71 to move the X-ray detecting portion 72. A support portion 73 may be positioned at a lower side of the body portion 71 of the stand 70 to support the body portion 71.

The X-ray detecting portion 72 may be movable in an automatic manner and a manual manner. The X-ray detecting portion 72 may include a motor (not shown) and a driving apparatus (not shown) to move the X-ray detecting portion 72.

The position recognition member 80 may be configured to recognize the position of the X-ray detecting portion 72. A bar code portion 81 may be provided at a portion of the body portion 71 that faces the X-ray detecting portion. A bar code reader portion 82 may be provided at a rear surface of the X-ray detecting portion 72 to recognize the bar code portion 81. Accordingly, as the X-ray detecting portion 72 moves upward and downward, the bar code reader portion 82 may scan the bar code portion 81 to recognize the position of the X-ray detecting portion 72.

The X-ray detecting portion 72 may be provided at the rear surface thereof with a protrusion 83 that is provided to have the bar code reader portion disposed thereon. However, the bar code reader portion 82 may be provided inside the rear portion of the X-ray detecting portion 72. In the case where the bar code reader portion 82 is provided inside the rear portion of the X-ray detecting portion 72, the X-ray detecting portion 72 may be formed using transparent material.

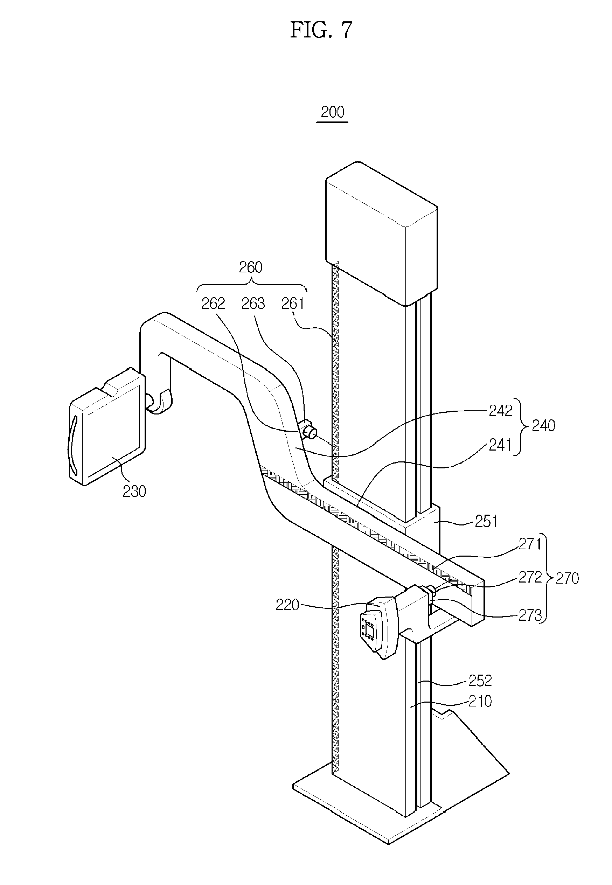

FIG. 7 is a view illustrating a medical imaging apparatus in accordance with one or more embodiments. A medical imaging apparatus 200 illustrated in FIG. 7 may be provided in a U-arm type that is able to obtain an image by use of a single X-ray detecting portion 230. The U-type medical imaging apparatus may have a superior spatial efficiency by having an X-ray generating portion 220 and the X-ray detecting portion 230 incorporated in a single apparatus.

The U-type medical imaging apparatus 200 may include a body portion 210 vertically disposed while being fixed to the ground surface, an X-ray generating portion 220 to generate and transmit X-rays, an X-ray detecting portion 230 to detect the X-rays transmitted from the X-ray generating portion 220, and an arm portion 240 to connect the X-ray detecting portion 230 to the X-ray generating portion 220.

The body portion 210 may be provided with a motor that has the arm portion 240 coupled thereto to enable a vertical movement and rotation of the arm portion 240.

The X-ray detecting portion 230 may be disposed at one side of the arm portion 240, and the X-ray generating portion 220 may be disposed at the other side of the arm portion 240. The arm portion 240 may include a first arm portion 241 coupled to the body portion 210, and a second arm portion 242 extending to the X-ray detecting portion 230 from the first arm portion 241. The first arm portion 241 and the second arm portion 242 may be rotatably provided to adapt to a region of the object that is to be photographed. The arm portion 240 may be provided with a motor to vertically move the X-ray generating portion 220 and a motor to rotate the X-ray detecting portion 230.

The arm portion 240 may be vertically movable along the body portion 210. A first moving portion configured to move the arm portion 240 may be provided at the body portion 210.

The first moving portion may include a rail 252 and a lifting member 251 coupled to the rail 252.

The X-ray generating portion 220 may be installed so as to move lengthways along the second arm portion. A second moving portion (not shown) may be formed at the second arm portion 242 to which the X-ray generating portion 220 is coupled. The X-ray generating portion 220 may be movable to adapt to the region of the object that is to be photographed.

A position recognition member may be provided on at least one of the first moving portion and the second moving portion. A first position recognition member 260 and a second position recognition member 270 may be provided at the first moving portion and the second moving portion, respectively.

The first position recognition member 260 may be provided at one side of the body portion 210 and at one side of the second arm portion 242. A first bar code portion 261 may be vertically provided at the one side of the body portion 210, and a first bar code reader portion 262 to recognize the first bar code portion 261 may be provided at the one side of the second arm portion 242 facing the body portion 210. Accordingly, as the arm portion 240 vertically moves along the first moving portion of the body portion 21, the first bar code reader portion 262 provided at the second arm portion 242 may scan the first bar code 261 provided at the body portion 210 to possibly recognize the position of the arm portion 240.

The second position recognition member 270 may include a second bar code portion 272 provided at a side of the first arm portion 251, to which the X-ray generating portion 220 is coupled, and a second bar code reader portion 272 provided at a rear side of the X-ray generating portion 220 facing the first arm portion 241. As the X-ray generating portion 220 moves lengthways along the first arm portion 241, the second bar code portion 271 of the first arm portion 241 may be recognized by the second bar code reader portion 272 disposed at the rear side of the X-ray generating portion 220, so that the position of the X-ray generating portion 220 may be recognized.

The first recognition member 260 and the second recognition member 270 may include a protrusion 263 and a protrusion 273, respectively, such that the bar code reader portions 262 and 272 recognize the bar code portions 261 and 272, respectively.

The first bar code reader portion 262 may be positioned inside the second arm portion 242. In this case, one side of the second arm portion 24 transmitting light of the first bar code reader portion 262 may be formed using transparent material. The second barcode reader portion 272 may be positioned inside the rear portion of the X-ray generating portion 220. In this case, the rear portion of the X-ray generating portion 220 may be formed using transparent material.

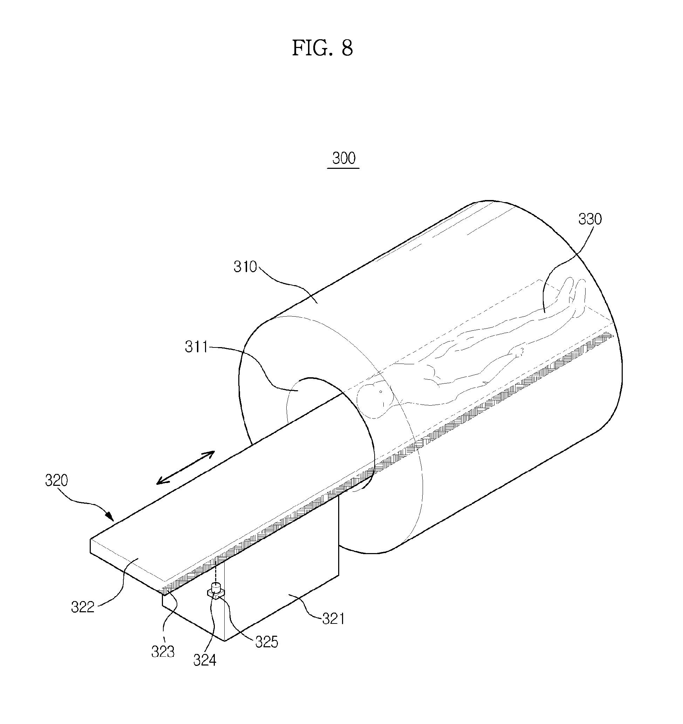

FIG. 8 is a view illustrating a medical imaging apparatus in accordance with one or more embodiments.

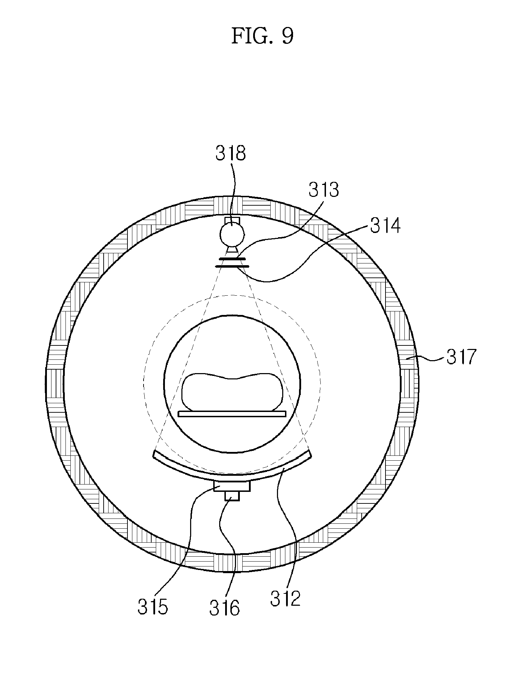

Referring to FIGS. 8 and 9, the medical imaging apparatus implemented as a Computed Tomography imaging apparatus 300, according to one or more embodiments. The CT imaging apparatus 300 is designed to transmit X-rays in a plurality of directions and reconstruct an image through a computer. The CT imaging apparatus 300 may include a gantry 310 provided with an X-ray generating portion 318 and an X-ray detecting portion 315, and a table 320 to accommodate and transport an object 330.

The gantry 310 may be provided at a center thereof with a cylindrical tunnel 311 into which the object 330 may be inserted. The X-ray generating portion 318 and the X-ray detecting portion 315 may be provided inside the gantry 310 while being disposed opposite each other. In addition, each of the X-ray generating portion 318 and the X-ray detecting portion 315 may be disposed in a predetermined position where X-rays are introduced to the object 330 inserted into the tunnel 311 of the gantry 310 and the X-rays may be detected.

The gantry 310 is rotated by a gantry driving member (not shown) on the object 330 while surrounding the tunnel 311. The gantry driving member may control the rotation speed of the gantry 310 at a constant speed, and may horizontally drive the gantry 310 back and forth.

A collimator 313 may be provided at a front side of the transmission direction of X-rays of the X-ray generating portion 318. The collimator 313 may allow X-rays to be transmitted in a fan shape toward the object 330 without being scattered in different directions. The collimator 313 as such may be disposed at a front side of the X-ray detecting portion 315. The collimator 313 may detect X-rays only at a region of interest.

An X-ray filtering portion 314 may be disposed at a front side of the collimator 313. The X-ray filtering portion 314 may filter X-rays in a predetermined range of energy band.

The table 320 may be provided with a transport portion 322 to transport the object 330 to the inside the gantry 310. A support portion 321 may be provided at a lower side of the table 320 to support the table 320. The support portion 322 may be disposed at an upper side of the table 320. The transport portion 322 may horizontally drive the tunnel 311 of the gantry 310 back and forth such that an examination region of the object 330 is disposed between the X-ray generating portion 318 and the X-ray detecting portion 315.

As the object 330 is inserted into the tunnel 311 of the gantry 310 by the transport portion 322 of the table 320, the gantry 310 may rotate around the object 330 by a predetermined angle or 360 degrees at the same time when cross-sectional images at various angles are obtained by the X-ray generating portion 318 and the X-ray detecting portion 315.

First position recognition members 315, 316, and 317 may be provided at an inner side of the gantry 310. A first moving portion to rotate the X-ray generating portion 318 and the X-ray detecting portion 315 may be provided at an inner side of the gantry 310. The first position recognition members 315, 316, and 317 may include a first barcode portion 317 that may surround the inner side of the gantry 310 in the form of a circle. The first bar code portion 317 may be provided to recognize the position of at least one of the X-ray generating portion 318 and the X-ray detecting portion 315 that may be provided at an inner wall of the gantry 310.

A first bar code reader portion 316 to recognize the first bar code portion may be disposed at a rear side of the X-ray generating portion 318 or a rear side of the X-ray detecting portion 315 that rotate. The first barcode reader portion 316 may be disposed opposite the inner wall of the gantry 310 where the first barcode portion 317 may be positioned.

The table 320 may be provided with second position recognition members 323, 324, and 325. A second moving portion may be provided at a coupling surface where the support portion 321 is coupled to the transport portion 322 that may be movable to the inside of the gantry 310. A second bar code portion 323 may be provided at a lower side of the transport portion 322, and a second bar code reader portion 324 may be provided at one side of the support portion 321. The second bar code reader portion 324 may be disposed opposite the second bar code portion 323. Accordingly, as the transport portion 322 is introduced to the inside of the gantry 310, the second bar code reader portion 324 may recognize the second bar code portion 323, which may be provided at a lower side of the transport portion 322, so that the movement of the transport portion 322 may be recognized.

Similar to the above described embodiment, the position recognition members illustrated in FIGS. 8 and 9 may also include protrusions 315 and 325. In a case where the bar code reader portion is positioned at an inner side of the gantry, the gantry may be formed using transparent material that allows light to pass therethrough.

While aspects of the present invention has been particularly shown and described with reference to differing embodiments thereof, it should be understood that these embodiments should be considered in a descriptive sense only and not for purposes of limitation. Descriptions of features or aspects within each embodiment should typically be considered as available for other similar features or aspects in the remaining embodiments. Suitable results may equally be achieved if the described techniques are performed in a different order and/or if components in a described system, architecture, device, or circuit are combined in a different manner and/or replaced or supplemented by other components or their equivalents.

Thus, although a few embodiments have been shown and described, with additional embodiments being equally available, it would be appreciated by those skilled in the art that changes may be made in these embodiments without departing from the principles and spirit of the invention, the scope of which is defined in the claims and their equivalents.

## Claims

1. A medical imaging apparatus comprising:
an X-ray generating portion configured to generate X-rays and transmit the X-rays;
an X-ray detecting portion configured to detect the X-rays transmitted from the X-ray generating portion;
a moving portion provided on at least one of the X-ray generating portion and the X-ray detecting portion to move the at least one of the X-ray generating portion and the X-ray detecting portion; and
a position recognition member comprising a bar code portion configured to recognize a position of the at least one of the X-ray generating portion and the X-ray detecting portion, which move along the moving portion, and a bar code reader portion to recognize the bar code portion.

2. The medical imaging apparatus of claim 1, wherein the bar code reader portion is formed on a protrusion that protrudes from at least one portion of the medical imaging apparatus, the at least one portion facing the bar code portion.

3. The medical imaging apparatus of claim 1, wherein the bar code reader portion is formed at an inside of at least one portion of the medical imaging apparatus, the at least one portion facing the bar code portion.

4. The medical imaging apparatus according to any of claims 1-3, wherein the X-ray generating portion comprises a direction adjusting portion configured to adjust a direction in which the X-ray is transmitted toward an object;
wherein the direction adjusting portion comprises a guide rail and a moving carriage that is disposed at a lower side of the guide rail to move along the guide rail; and
wherein the moving portion is provided on the guide rail, the guide rail comprises a first guide rail and a second guide rail, the bar code portion is positioned on at least one of the first guide rail and the second guide rail, and the bar code reader portion to recognize the bar code portion is provided at an upper side of the moving carriage, which faces the at least one of the first guide rail and the second guide rail.

5. The medical imaging apparatus of claim 4, further comprising a driving apparatus configured to support one of the first guide rail and the second guide rail against a remaining one of the first guide rail and the second guide rail while driving the remaining one of the first guide rail and the second guide rail with respect to the one of the first guide rail and the second guide rail; and
wherein each of the first guide rail and the second guide rail is provided in one pair, a first bar code portion is provided at one side of the first guide rail, a first bar code reader portion to recognize the first bar code portion is provided at one side of the moving carriage, a second bar code portion is provided at one side of the second guide rail, and a second bar code reader portion to recognize the second bar code portion is provided at an other side of the moving carriage.

6. The medical imaging apparatus according to any of the foregoing claims, further comprising a table configured to have the object disposed thereon, the table comprising a table top on which the object is disposed and a support to support the table top;
wherein the X-ray detecting portion is provided at a lower side of the table top;
wherein the moving portion is disposed at the lower side of the table top to move the X-ray detecting portion; and
wherein the bar code portion is provided at an edge of the table, and the bar code reader portion is provided at one side of the X-ray detecting portion.

7. The medical imaging apparatus according to any of the foregoing claims, wherein the X-ray detecting portion is comprised on a stand;
said stand further comprising a body portion; and
the X-ray detecting portion being movably coupled to the body portion.

8. The medical imaging apparatus according to any of the foregoing claims, wherein the moving portion is mounted on the body portion to move the X-ray detecting portion upward and downward, the bar code portion being provided on one surface of the body portion facing the X-ray detecting portion, and the bar code reader portion being provided at a rear surface of the X-ray detecting portion.

9. The medical imaging apparatus according to any of the foregoing claims, further comprising:
an arm portion enabling the X-ray detecting portion and the X-ray generating portion to be installed in one apparatus.

10. The medical imaging apparatus of claim 9, further comprising a body portion configured to support the arm portion, which connects the X-ray detecting portion to the X-ray generating portion.
wherein the arm portion comprises a first arm portion coupled to the body portion, and a second arm portion extending from the first arm portion to the X-ray detecting portion; and
wherein the bar code portion is provided at one side of the body portion facing the second arm portion, and the bar code reader portion is provided at one side of the second arm portion facing the body portion.

11. The medical imaging apparatus according to claim 9 or 10, further comprising a body portion configured to support the arm portion, which connects the X-ray detecting portion to the X-ray generating portion;
wherein the arm portion comprises a first arm portion coupled to the body portion, and a second arm portion extending from the first arm portion to the X-ray detecting portion; and
wherein the bar code portion is provided at one surface of the first arm portion to which the X-ray generating portion is coupled, and the bar code reader portion is provided at a rear side of the X-ray generating portion.

12. A medical imaging apparatus comprising:
a gantry provided at a center thereof with a round tunnel to accommodate an object while being configured to rotate around the object, the gantry comprising an X-ray generating portion to generate X-rays at an inner side of the gantry and transmit the X-rays, an X-ray detecting portion to detect the X-rays received from the X-ray generating portion, and a position recognition member comprising a first bar code portion to recognize coordinates of the X-ray generating portion and a first bar code reader portion to recognize the first bar code portion;
a table having the object disposed thereon, and comprising a transport portion to transport the object to an inside of the gantry and a support potion to support the table; and
a moving portion provided on at least one of the table and the inner side of the gantry.

13. The medical imaging apparatus of claim 12, wherein the first bar code portion surrounds the inner side of the gantry in a circle, and the first bar code reader portion is provided at a rear side of the X-ray detecting portion that faces an inner wall of the gantry.

14. The medical imaging apparatus according to claim 12 or 13, wherein the first bar code portion surrounds the inner side of the gantry in a circle, and the first bar code reader portion is provided at a rear side of the X-ray generating portion that faces an inner wall of the gantry.

15. The medical imaging apparatus according to any of claims 12-14, wherein the table comprises a second bar code portion provided at one side of the transport portion to recognize a position of the object, and a second bar code reader portion provided at the support portion to recognize the second bar code portion.
